# EUROPEAN PATENT APPLICATION

(11) **EP 4 541 355 A1**
(43) Date of publication of application: **23.04.2025**
(21) Application number: 24207390.6
(22) Date of filing: 18.10.2024
(51) Int. Cl.: A61K 31/4706, A61K 33/30, A61P 3/00, A61P 43/00

(54) **SPECIFIC TETRADENTATE COPPER CHELATORS FOR USE IN THE TREATMENT OF WILSON'S DISEASE**

(30) Priority: 20.10.2023 WO PCT/CN2023/125744
(71) Applicant: Guangdong University of Technology, Guangzhou, Guangdong 510006 (CN); The French National Centre for Scientific Research (Centre National de la Recherche Scientifique), 75794 Paris (FR)
(72) Inventor: LIU, Yan, Wuhan (CN); ZHU, Yingshan, Wuhan (CN); TANG, Ying, Wuhan (CN); HUANG, Lan, Wuhan (CN); ANNE, Robert, Paris (FR); BERNARD, Meunier, Paris (FR)
(74) Representative: Murgitroyd & Company

(57) **Abstract**

A method for treating Wilson's disease with tetradentate copper chelators therapy is provided. Specific copper chelators of the TDMQ tetradentate series are highly efficient for the treatment of Wilson's disease murine model. The concentration of copper in liver of "toxic milk" TX mice decreased and the fecal excretion of copper increased with the treatment with TDMQ20. Both effects are dose-dependent. Pharmacological data obtained on this TX mouse model with TDMQ20 strongly support the selection of this type of ligand as drug-candidate for this genetic disease.

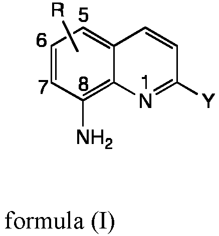

## Description

### FIELD of APPLICATION

The present invention is related to the field of Wilson's disease. More specifically, the present invention provides methods useful for the treatment of Wilson's disease.

### BACKGROUND OF THE INVENTION

Wilson's disease (WD) is a genetic disease due to mutations on the *Atp7b* gene coding for a copper carrier protein in charge of the excretion of the excess of copper from liver. In the world, this autosomal recessive disorder of copper metabolism concerns 1 person over 30,000. The protein ATP7B transports the excess of copper from liver to the bile and then to the feces. When this copper carrier is deficient, the excess of copper is released in the bloodstream and slowly eliminated in urine, generating an accumulation of copper ions which causes chronic hepatitis and cirrhosis. Finally, an excess of copper is detected in the brain, leading to neurological disorders (A. Lucena-Valera et al., World J. Hepatol., 13 (6) 634-649, 2021). Without treatment, WD can be fatal.

Current medical treatments involve the use of copper chelators to increase the excretion of copper. The first line drug for WD treatment is currently *D*-penicillamine (DPA) by oral administration at 750-1500 mg/day in adults. Several adverse effects of DPA have been identified with time: nephrotoxicity, aplasia, retinitis and neurological deterioration.

The second drug used for WD is trientine (triethylene-tetramine) with oral doses ranging from 900 to 2700 mg/day in adults. Severe adverse effects are also observed with time: bone marrow depression, anemia, skin rash and hemorrhagic gastritis.

There is a real medical need for a specific copper chelator able to efficiently regulate the copper excess at lower doses than the ones used for DPA or trientine. The main drawback of DPA and trientine is their lack of metal selectivity, since they have similar affinities for copper and zinc. Our research group designed a new series of tetradentate ligands based on an 8-aminoquinoline entity, named TDMQ, which are highly selective for copper compared with other metal ions like zinc, and have been qualified as drug-candidates for the treatment of Alzheimer's disease (for patents, see Y Liu et al., China (GDUT-CNRS), 27 May 2016, n° 201610369550.X, Patent number US 10,807,957 B2. Approved on October 20, 2020. Canada, patent number 3 025 406, approved on June 1st, 2021. Japan, patent number 6889825, approved on May 26th, 2021. Europe, patent application in final discussion). Since these TDMQ ligands have a high bioavailability (L. Huang et al., Pharmaceutics, 14, 2691, 2022), we decided to evaluate their activity as drug-candidates for the treatment of Wilson's disease. In addition, because of the very weak affinity of these TDMQ ligands for zinc, it should be noted that these molecules will be compatible with the co-treatment with zinc salts that is also used in WD therapy to reduce the absorption of copper in the intestinal track (see A. Lucena-Valera *et al.,* cited above).

The chemical syntheses of TDMQ ligands have been reported in the patents mentioned above (for an article, see W. Zhang et al., ChemMedChem, 13, 864-704, 2018). Among all various TDMQ ligands, TDMQ20 has been selected as the best drug-candidate because of its suitable pharmacological profile evidenced in mouse AD-models (see J. Zhao et al., ACS Chem. Neurosci., 12, 140-149, 2021).

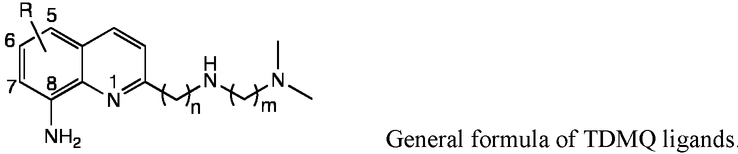

For TDMQ20: R = 5,7-dichloro-, n = m = 2.

### SUMMARY OF THE INVENTION

In view of the above-mentioned medical need for a more efficient copper chelator with reduced side effects for the treatment of Wilson's disease, the present invention concerns specific tetradentate copper chelators that are effective in the *in vivo* treatment by oral route of a mouse model of the Wilson's disease.

The invention concerns the use of specific copper chelators of the tetradentate series named TDMQ in the treatment of Wilson's disease. In particular, the invention provides evidences that TDMQ20 has a better pharmacological activity at lower doses by oral administration than D-penicillamine (DPA), a current drug used in the treatment of the Wilson's disease.

TDMQ20 is better than D-penicillamine to improve the normal/non-pathological pathway of copper excretion.

The copper concentration in the liver of mice treated by the three different doses of TDMQ20 decreased when the drug dose increased, indicating that the pharmacological effect of TDMQ20 is dose-dependent. At 50 mg/kg/day, TDMQ20 (TDMQ-H) is more efficient in decreasing the copper concentration in liver than D-penicillamine (DPA) at 200 mg/kg/day which is considered at the moment one of the best possible treatments for WD.

The copper content in feces is significantly higher at 25 or 50 mg/kg/day with TDMQ20 compared to untreated WD mice or to mice treated with DPA at 200 mg/kg/day, indicating an increased fecal elimination of copper upon treatment by TDMQ20. This effect of TDMQ20 is dose-dependent.

The copper concentration in serum of WD mice treated with TDMQ20 increased when the TDMQ20 dose increased. The highest dose of TDMQ20 (TDMQ20-H, 50 mg/kg/day) corresponds to the highest copper concentration in serum, confirming the ability of the drug to extract copper from the liver. The copper concentration in the serum of WD mice treated with DPA (200 mg/kg/day) is not significantly higher than that of untreated WD mice, and far below that observed for mice treated with TDMQ20 at 50 mg/kg. The copper concentration in urine of WD mice was significantly higher than that of controls. Upon treatment by TDMQ20 at 50 mg/kg, copper concentration in urine was significantly increased compared to WD mice by a factor of 1.8, indicating a significant improvement of urinary excretion of copper.

The copper concentration was also measured in the brain of mice. Data indicated that the copper concentration was similar in the brain of controls and WD mice, and that there is no increase of copper concentration in WD mice treated with TDMQ20 whatever the used dose. Since neurological disorders are often observed in WD patients, it was important to check that TDMQ20 was unable to carry copper to the brain.

The concentration of ceruloplasmin (CP), that contains the most part of blood copper, was measured in the serum of treated WD mice. As expected, it was significantly lower than that of control mice. The level of CP was significantly increased in mice treated with TDMQ20 at 25 or 50 mg/kg (TDMQ20-M and TDMQ20-H, respectively) compared to WD mice. The CP level was equal (25 mg/kg) or higher (50 mg/kg) than that observed in control mice. Since the level of CP is considered as a significant parameter in the clinical control of human Wilson's disease, the reversal of the CP impairment upon treatment by TDMQ20 confirms that this drug has a very good profile as drug-candidate for the treatment of Wilson's disease.

Other differentially expressed proteins (DEPs) were screened at *p* < 0.05 in the treated WD mouse group compared to the control group. Fourteen proteins were found down-regulated in the liver of WD mice compared to controls, while 29 hepatic proteins were found up-regulated. In the serum of WD mice, five proteins were down-regulated and five were up-regulated. After treatment by TDMQ20 at 25 or 50 mg/kg (TDMQ20-M and TDMQ20-H groups, respectively), all these serum and hepatic DEPs were found reversed to normal values.

### BRIEF DESCRIPTION OF THE DRAWINGS

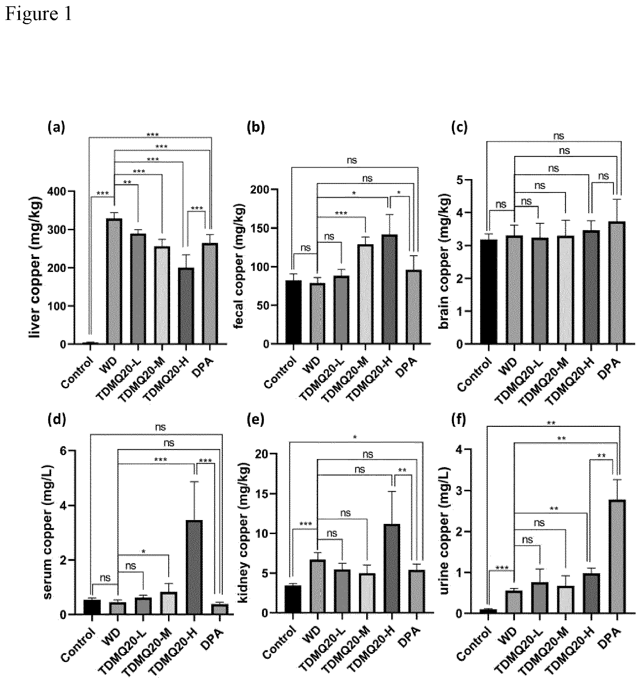

FIG. 1 shows the effect of concentration of copper in liver (a), feces (b), brain (c), serum (d), kidney (e) and urine (f) of control mice, TX mice (WD) and TX mice orally treated with 3 different dosages of TDMQ20 (12. 5 mg/kg, 25 mg/kg or 50 mg/kg, L, M and H respectively) or by DPA (200 mg/kg). Data analysis was carried out using SPSS Statistics v. 26. *p* > 0.05 differences were considered not significant (ns), * *p <* 0.05, ** *p <* 0.01 and *** *p <* 0.001, n=6. The scheme was generated by using the Prism GraphPad 8.0.2. software.

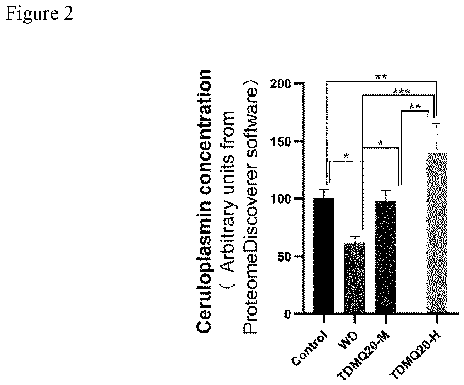

FIG. 2 shows that the effect of different concentration of serum ceruloplasmin labeled with TMT and detected by LC-MS/MS. * p < 0.05, ** p < 0.01 and *** p < 0.001.

### DEFINITIONS

WD is the short name for Wilson's disease.

DPA is the short name for *D*-penicillamine, a current metal chelating agent used for the treatment of Wilson's disease.

TDMQ refers to a series compound of the formula (I).

The term "specific tetradentate copper chelators" refers to a series compound of the formula (I).

TDMQ-L, TDMQ-M, TDMQ-H represent different doses of TDMQ, 12.5, 25.0, 50.0 mg/kg, respectively.

### DETAILED DESCRIPTION OF THE ILLUSTRATED EMBODIMENTS

### EMBODIMENT 1: Pharmacological activity of TDMQ ligands on a mouse model of Wilson's disease

The selected drug-candidate TDMQ20 has been evaluated on the homozygous toxic-milk mouse model (TX model), a regular murine model of Wilson disease, suitable for evaluation of drug-candidates (for a review, see E. Reed et al., J. Neurochem., 146, 356-373, 2018). TX mice are bearing a naturally occurring point mutation on ATP7B resulting in a methionine changed for a valine, that causes the loss of copper transportation by this protein. C57BL/6 mice have no mutation on ATP7B and were used as control mice in the evaluation of TDMQ20. TX mice were provided by Sun Yat-Sen University (Guangzhou) and C57BL/6 mice by the Guangdong experimental animal center (Guangzhou) and all experiments were performed according to protocols approved by the Welfare Laboratory Animals Committee of the Guangdong University of Technology (approval n°GDUTXS2022083 dated on 3 August 2022), in the framework of the 3Rs principle.

8-Week-old TX mice (WD) and 8-week-old C57BL/6 mice (Control) were used. TX mice (as WD, TDMQ-L, TDMQ-M, TDMQ-H groups, respectively) received the drug TDMQ20 by intragastric gavage twice a day for 14 consecutive days (at 0, 12.5, 25.0, 50.0 mg/kg in NaCl 0.9%). On the same schedule, C57BL/6 mice (as control group) received NaCl 0.9%. Treatment of WD mice with DPA (200 mg/kg) was used as comparison (DPA group). Six groups of 6 mice were used. The animal groups and treatments are summarized in Table 1.

**Table 1**

| Groups A to F | Mouse type | Drug | Drug dose (mg/kg/day) | Number of mice | Number of mice | |
|---|---|---|---|---|---|---|
| | | | | | male | female |
| (A): Control | C57BL/6 mice | 0.9% NaCl | 0 | n = 6 | 3 | 3 |
| (B): WD | TX mice | 0.9% NaCl | 0 | n = 6 | 3 | 3 |
| (C): TDMQ20-L | TX mice | TDMQ20 in 0.9% NaCl | 12.5 | n= 6 | 2 | 4 |
| (D): TDMQ20-M | TX mice | | 25 | n = 6 | 3 | 3 |
| (E): TDMQ20-H | TX mice | | 50 | n = 6 | 2 | 4 |
| (F): DPA* | TX mice | DPA in 0.9% NaCl | 200 | n = 6 | 3 | 3 |

| | | | | | | |
|---|---|---|---|---|---|---|
| *Definition of the six different groups (A to F) of mice with the different drug treatments. The D-penicillamine dose was defined according to J. W. Zhang et al., Biochem. Biophys. Res. Commun., 458, 82-85, 2015. Since the gavage was performed twice a day, half of the doses indicated in this Table was given at 12 h interval. * DPA stands for D-penicillamine. | | | | | | |

At the end of the treatment period, mice (10-week old) were placed in metabolic cages for 12 h in order to collect urine and feces. At the end of this 12 h-period, mice were anesthetized using 2.5 % Avertin diluted in 0.9 % NaCl at a dose of 125 mL/10 g body weight and sacrificed. Cardiac blood was collected and left at room temperature for 30 minutes before centrifugation at 4 °C at 3000 r/min for 10 minutes to obtain serum samples. During this 30-min period, liver, kidney, brain and serum were also collected. Copper concentrations in urine and feces, serum, liver, kidney and brain were determined by inductively coupled plasma mass spectrometry (ICP-MS). Data analysis was carried out using SPSS Statistics software (version 26). Data are provided as mean values ± standard error of the mean (SEM). Statistical analyses were performed using one-way ANOVA for liver, feces and urine, and by non-parametric test for serum, kidney, and brain. Differences with *p >* 0.05 were considered as no significance (ns), * *p <* 0.05, ** *p* < 0.01, and *** *p* < 0.001, n = 6. Graph presentations were drawn with Prism GraphPad 8.0.2.

### Pharmacological results are reported in Table 2 and in Fig.1.

**Table 2**

| | **Cu content as mean value of 6 mice ± SEM value** | | | | | |
|---|---|---|---|---|---|---|
| | **Control** | **WD** | **TDMQ20-L** | **TDMQ20-M** | **TDMQ20-H** | **DPA** |
| **Liver (mg/kg)** | 4.3 ± 0.4 | 328 ± 6 | 290 ± 4 | 256 ± 8 | 200 ± 14 | 265 ± 9 |
| **Feces (mg/kg)** | 82 ± 4 | 79 ± 3 | 88 ± 3 | 129 ± 4 | 142 ± 10 | 96 ± 7 |
| **Serum (mg/L)** | 0.54 ± 0.02 | 0.46 ± 0.03 | 0.63 ± 0.04 | 0.83 ± 0.12 | 3.5 ± 0.6 | 0.39 ± 0.03 |
| **Kidney (mg/kg)** | 3.5 ± 0.1 | 6.7 ± 0.4 | 5.5 ± 0.3 | 5.0 ± 0.4 | 11.0 ± 1.7 | 5.4 ± 0.3 |
| **Urine (mg/L)** | 0.10 ± 0.01 | 0.56 ± 0.02 | 0.76 ± 0.13 | 0.67 ± 0.10 | 0.98 ± 0.05 | 2.8 ± 0.2 |
| **Brain (mg/kg)** | 3.2 ± 0.1 | 3.3 ± 0.1 | 3.2 ± 0.2 | 3.3 ± 0.2 | 3.5 ± 0.1 | 3.7 ± 0.3 |

| | | | | | | |
|---|---|---|---|---|---|---|
| *Concentration of copper in control mice, TX mice (WD), and TX mice treated with TDMQ20 or DPA as mean values for 6 mice of each group ± SEM, calculated using the SPSS Statistics software, v. 26. Results are in mg/kg for solids (liver, feces, kidney and brain) and in mg/L for fluids (urine and serum). | | | | | | |

### Comments on the results

1) The copper concentration in the liver (Fig. 1a) of mice treated by the three different doses of TDMQ20 decreased when the drug dose increased, indicating that the pharmacological effect of TDMQ20 is dose-dependent. At 50 mg/kg/day, TDMQ20 (TDMQ-H) is more efficient in decreasing the copper concentration in liver than D-penicillamine (DPA) at 200 mg/kg/day which is considered at the moment the best possible treatment for WD. It should be noted that the concentration of copper in liver of mice treated at 25 mg/kg/day (256 mg/kg) is close to the value obtained with DPA at 200 mg/kg/day (265 mg/kg). The dose of DPA is 8 times the dose of TDMQ20 for the same result, indicating the better efficiency of TDMQ20.
2) The copper content in feces (Fig. 1b) is significantly higher at 25 or 50 mg/kg/day with TDMQ20 compared to untreated WD mice or to mice treated with DPA at 200 mg/kg/day, indicating an increased fecal elimination of copper upon treatment by TDMQ20. This effect of TDMQ20 is dose-dependent.
3) The copper concentration in serum of WD mice treated with TDMQ20 was increased when the TDMQ20 dose was increased (Fig.1d). The highest dose of TDMQ20 (TDMQ20-H, 50 mg/kg/day) corresponds to the highest copper concentration in serum, confirming the ability of the drug to extract copper from the liver. The copper concentration in the serum of WD mice treated with DPA (200 mg/kg/day) is not significantly higher than that of untreated WD mice, and far below that observed for mice treated with TDMQ20 at 50 mg/kg. These data confirm that TDMQ20 is able to transfer copper to endogenous copper carriers *in vivo.*
4) The concentration of copper in kidney of WD mice after treatment by TDMQ20 at 50 mg/kg, was higher than that of WD mice by a factor of 1.64 (Fig.1e). This ratio is very similar to concentration ratio TDMQ20-H/WD of 1.75 observed in urine (see Fig.1f and data of Table 2). These data indicate an important excretion of copper via kidney when WD mice are treated with TDMQ20. This excretion mediated by TDMQ20 is also significantly higher than that observed in WD mice treated with DPA at 200 mg/kg.
5) As expected, the copper concentration in urine of WD mice was significantly higher than that of controls (Fig.1f). Upon treatment by TDMQ20 at 50 mg/kg, copper concentration in urine was significantly increased compared to WD mice by a factor of 1.8, indicating a significant improvement of urinary excretion of copper.
6) The copper concentration was also measured in the brain of mice (Fig.1c). Data indicated that the copper concentration was similar in the brain of controls and WD mice, and that there is no increase of copper concentration in WD mice treated with TDMQ20 whatever the drug dose. Since neurological disorders are often observed in WD patients, it was important to check that TDMQ20 was unable to carry copper to the brain.

**Conclusion.** The treatment of the murine model of Wilson disease TX mice by TDMQ20, resulted in a significant decrease of the copper concentration in the liver, and significant increase of fecal excretion of copper compared to WD mice. This dose-dependent effect indicates that treatment with TDMQ20 significantly improves the normal/non-pathological pathway of copper excretion, which is not the case in WD mice treated with the reference drug DPA at 200 mg/kg.

### EMBODIMENT 2: Differentially expressed hepatic and serum proteins in WD mice returned to normal levels after TDMQ20 administration

In order to confirm the positive role of TDMQ20 in the treatment of TX mice, we performed proteomic experiments. Samples from three mice of each group were depicted in Table 1, The samples were treated with TMT (Tandem Mass Tag) and detected by quantitative LC-MS/MS, then the data were analyzed with ProteomeDiscoverer 2.4.1.15 (ThermoFisher Scientific). Confidence proteins were screened according to the criteria of Sequest program score greater than 0 and number of unique peptides greater than or equal to 1, and blank values were removed.

### Ceruloplasmin

The concentration of ceruloplasmin (CP), that contains the most part of blood copper, was measured by TMT LC-MS/MS in the serum of WD mice. As expected, it was significantly lower than that of control mice (p < 0.05, Fig.2). The level of CP was significantly increased in mice treated with TDMQ20 at 25 or 50 mg/kg (TDMQ20-M and TDMQ20-H, respectively) compared to WD mice. The CP level was equal (25 mg/kg) or higher (50 mg/kg) than that observed in control mice. Since the level of CP is considered as a significant parameter in the clinical control of human Wilson's disease, the reversal of the CP impairment upon treatment by TDMQ20 confirms that this drug has a very good profile as drug-candidate for the treatment of Wilson's disease.

### Other proteins

Other differentially expressed proteins (DEPs) were screened at *p* < 0.05 in the WD mouse group compared to the control group. Fourteen proteins were found down-regulated in the liver of WD mice compared to controls, while 29 hepatic proteins were found up-regulated. In the serum of WD mice, five proteins were down-regulated and five were up-regulated. After treatment by TDMQ20 at 25 or 50 mg/kg (TDMQ20-M and TDMQ20-H groups, respectively), all these serum and hepatic DEPs were found reversed to normal values, with a TDMQ20-H/control ratio close to 1. Results are reported in Table 3 and 4 for hepatic and serum proteins, respectively.

**Table 3**

| **Protein** | **WD/ control** fold change | **TDMQ20-M/control** fold change | **TDMQ20-H/control** fold change |
|---|---|---|---|
| ***a) Liver DEPs down-regulated in WD with respect to control*** | | | |
| Aspartate-tRNA ligase, cytoplasmic (Dars1) | 0.77 | 1.03 | 1.13 |
| Progranulin (Gm) | 0.77 | 1.05 | 1.04 |
| Putative phospholipase B-like 2 (Plbd2) | 0.74 | 0.98 | 0.99 |
| ATP-citrate synthase (Acly) | 0.80 | 0.86 | 0.97 |
| Ribosome biogenesis protein BOP1 (Bop1) | 0.77 | 1.08 | 0.97 |
| 2-Oxo-4-hydroxy-4-caboxy-5-ureidoimidazoline decarboxylase (Urad) | 0.77 | 0.93 | 0.96 |
| Charged multivesicular body protein 7 (Chmp7) | 0.80 | 1.04 | 0.94 |
| Nicotinamide/nicotinic acid mononucleotide adenylyltransferase 3 (Nmnat3) | 0.82 | 0.97 | 0.94 |
| Mesencephalic astrocyte-derived neurotrophic factor (Manf) | 0.80 | 0.94 | 0.91 |
| Triosephosphate isomerase (Tpi1) | 0.80 | 0.85 | 0.90 |
| Pyridoxine-5'-phosphate oxidase (Pnpo) | 0.75 | 1.01 | 0.90 |
| Desmin (Des) | 0.82 | 0.88 | 0.88 |
| Cytochrome P450 2C70 (Cyp2c70) | 0.73 | 0.85 | 0.88 |
| Alcohol deshydrogenase class-3 (Adh5) | 0.81 | 0.89 | 0.86 |

| ***b) Liver DEPs up-regulated in WD with respect to control*** | | | |
|---|---|---|---|
| Ribonuclease P protein subunit p30 (Rpp30) | 1.30 | 1.20 | 1.20 |
| Histone H2A.V | 1.40 | 1.14 | 1.19 |
| PRKC apoptosis WT1 regulator protein (Pawr) | 1.41 | 1.10 | 1.18 |
| Exosome complex exonuclease RRP44 (Dis3) | 1.24 | 1.16 | 1.00 |
| Histone H4 (H4c16) | 1.23 | 1.12 | 1.15 |
| Probable ATP-dependent RNA helicase DDX17 (Ddx17) | 1.23 | 1.01 | 1.11 |
| Mitochondrial outer membrane protein SLC25A46 (Slc25a46) | 1.25 | 1.10 | 1.11 |
| 28 kDa heat- and acid-stable phosphoprotein (Pdap1) | 1.24 | 1.15 | 1.10 |
| Cyclin-dependent kinase inhibitor 1B (Cdkn1b) | 1.28 | 1.13 | 1.10 |
| Protein scribble homolog (Scrib) | 1.40 | 1.07 | 1.09 |
| OCIA domain-containing protein 1 (Ociad1) | 1.28 | 1.14 | 1.08 |
| | | | |
| Histone H2B type 1-H (H2bc9) | 1.20 | 1.05 | 1.07 |
| Protein LYRIC (Mtdh) | 1.29 | 0.85 | 1.06 |
| Afadin (Afdn) | 1.20 | 1.07 | 1.05 |
| NADPH adrenodoxin oxidoreductase, mitochondrial (Fdxr) | 1.23 | 1.10 | 1.04 |
| Alpha-1-antitrypsin-1-2 (Serpina1b) | 1.50 | 1.09 | 1.04 |
| Alanine glyoxylate aminotransferase (Agxt) | 1.89 | 1.18 | 1.03 |
| Agmatinase, mitochondrial (Agmat) | 1.42 | 1.15 | 1.01 |
| SET nuclear proto-oncogene (Set) | 1.20 | 0.95 | 1.01 |
| Mitochondrial dicarboxylase carrier (S1c25a10) | 1.25 | 1.10 | 1.00 |
| Peroxisomal membrane protein-2 (Pxmp2) | 1.34 | 1.14 | 0.98 |
| Sideroflexin-5 (Sfxn5) | 1.28 | 1.06 | 0.96 |
| Protein Hook homolog 1 (Hook1) | 1.22 | 1.07 | 0.94 |
| Epsin-1 (Epn1) | 1.21 | 1.05 | 0.93 |
| AN1-type zinc finger protein 6 (Zfand6) | 1.35 | 1.09 | 0.91 |
| ATP synthase subunit a (Mtatp6) | 1.21 | 1.04 | 0.91 |
| Regucalcin (Rgn) | 1.64 | 1.07 | 0.91 |
| Zinc transporter 10 (Slc30a10) | 1.21 | 0.94 | 0.9 |
| Protein-glutamine gamma-glutamyltransferase K (Tgm1) | 1.31 | 0.90 | 0.84 |

| | | | |
|---|---|---|---|
| List of hepatic DEPs reversed after treatment by TDMQ20 at 0, 25 or 50 mg/kg (TDMQ20-M groups, respectively), a) DEPs down-regulated in WD, b) DEPs up-regulated in WD. | | | |

**Table 4**

| **Protein** | **WD/contro l** fold change | **TDMQ20-M/ctr l** fold change | **TDMQ20-H/ctr l** fold change |
|---|---|---|---|
| ***a) Serum DEPs down-regulated in WD with respect to control*** | | | |
| GrpE protein homolog 1, mitochondrial (Grpel1) | 0.81 | 0.95 | 1.04 |
| Alpha-aminoadipic semialdehyde synthase, mitochondrial (Aass) | 0.74 | 0.97 | 0.93 |
| Cystatin-C (Cst3) | 0.60 | 0.88 | 0.92 |
| Transgelin-2 (Tagln2) | 0.77 | 0.87 | 0.91 |
| Elongation factor 1-delta (Eef1d) | 0.76 | 0.85 | 0.88 |

| ***b) Serum DEPs up-regulated in WD with respect to control*** | | | |
|---|---|---|---|
| Ig heavy chain V region 1-72 (Ighv1-72) | 1.72 | 1.08 | 1.15 |
| | | | |
| Ribonuclease 4 (Rnase4) | 1.37 | 1.10 | 1.03 |
| Triokinase/FMN cyclase (Tkfc) | 1.31 | 1.07 | 0.95 |
| Tropomyosin alpha-3 chain (Tpm3) | 1.35 | 1.11 | 0.95 |
| Regucalcin (Rgn) | 1.49 | 1.06 | 0.88 |

| | | | |
|---|---|---|---|
| *List of serum DEPs reversed after treatment by TDMQ20 at 0, 25 or 50 mg/kg (TDMQ20-M groups, respectively), a) DEPs down-regulated in WD, b) DEPs up-regulated in WD. | | | |

These results indicate that the up- and down-regulated proteins in liver and serum of WD mice are reversed to normal values upon treatment by TDMQ20 at 25 or 50 mg/kg.

## Claims

1. A method for the treatment of Wilson's disease, comprising the administration of an effective amount of compound of the formula (I); 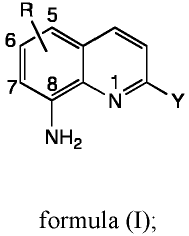 Where Y represents a group with the following formula: (CH₂)ₙ-NH-(CH₂)ₘ-N(CH₃)₂, n and m represent 1 or 2 or 3, R₅, R₆ and R₇ are the same or different, and independently represent a hydrogen atom, or a chlorine atom, or a fluorine atom, or a trifluoromethyl group.

2. A method as claimed in claim 1, wherein n = m = 2, R₅ = R₇ = Cl, R₆ = H is the preferred TDMQ molecule to manufacture a drug for the treatment of Wilson's disease.

3. The method of claim 1 for the treatment of Wilson disease by a compound of the formula (I), resulted in a significant decrease of the copper concentration in the liver, and significant increase both fecal and urinary elimination of copper.

4. The method of claim 1 does not increase the copper concentration in the brain during the treatment with a compound of the formula (I).

5. The method of claim 1 reverses to normal levels hepatic and serum proteins.

6. The method of claim 1 allows the serum ceruloplasmin level to return to normal values of controls after treatment by a compound of the formula (I).

7. The method of claim 1 for treating Wilson's disease either as monotherapy or as a part of polytherapy, including in association with zinc salts.

8. The therapeutic use of compounds from claims 1 or 2, wherein the drugs include acceptable salts of the TDMQ series. The hydrochloride form is the preferred one.
